# DEMANDE DE BREVET EUROPEEN

(11) **EP 0 592 266 A1**
(43) Date de publication de la demande: **13.04.1994**
(21) Numéro de dépôt: 93402297.1
(22) Date de dépôt: 21.09.1993
(51) Int. Cl.: A61B 17/60

(54) **Dispositif d'immobilisation d'une barre pour, notamment, ostéosynthèse ou arthrodèse**

(30) Priorité: 07.10.1992 FR 9212312
(71) Demandeur: Bouvet, Jean-Claude, F-91590 La Ferte Alais (FR)
(72) Inventeur: Bouvet, Jean-Claude, F-91590 La Ferte Alais (FR)
(74) Mandataire: Flavenot, Bernard

(57) **Abrégé**

La présente invention concerne les dispositifs d'immobilisation (1) d'une barre (2) par rapport à une partie osseuse (8).

Le dispositif selon l'invention se caractérise essentiellement par le fait qu'il comporte des moyens de fixation (3) comportant une tige (4) et une tête (5) à deux faces (6,7), la tige (4) étant apte à être reliée à la partie osseuse (8), des moyens (9) pour solidariser la barre (2) avec la tête (5) comprenant un cavalier (10) en forme sensiblement de "U" comportant une base (11) et deux branches (12,13) solidaires de la base (11), des moyens d'accrochage (14) de l'extrémité libre (15,16) des deux branches (12,13) avec la tête (5), et des moyens (17) pour exercer un couple de forces opposées entre le cavalier (10) et la tête (5) par l'intermédiaire de la barre (2) de façon à solidariser le cavalier (10), la tête (5) et la barre (2).

Application, notamment, à la réalisation d'ostéosynthèses ou d'arthrodèses rachidiennes.

## Description

La présente invention concerne les dispositifs d'immobilisation d'une barre par rapport à une partie osseuse en vue, par exemple, de réaliser une ostéosynthèse ou une arthrodèse, notamment rachidienne, c'est-à-dire les dispositifs qui permettent, au moyen d'au moins une barre et de moyens de fixation, de réaliser une liaison entre deux os tels que, par exemple, deux vertèbres d'une colonne vertébrale.

On connaît déjà différents types de dispositifs pour arthrodese rachidienne qui sont généralement constitués d'un ensemble de fixations aptes à être implantées dans les os à réunir et d'au moins une barre couplée solidairement à cet ensemble de fixations.

Un tel dispositif est par exemple décrit dans le Brevet DE-A-4 107 480. II comporte essentiellement une tige et une tête à deux faces opposées, un cavalier en forme sensiblement de "U" comprenant au moins une base et deux branches latérales solidaires de la base en l'une de leurs extrémités, deux crochets montés respectivement sur l'extrémité libre des deux branches latérales, latéralement à ces branches, vers l'intérieur du "U" et sensiblement en regard l'un de l'autre, et des moyens pour exercer un couple de forces opposées entre le cavalier et la tête par l'intermédiaire de la barre de façon à solidariser ensemble le cavalier, la tête et la barre.

Ce dispositif connu permet de fixer une barre pour obtenir une arthrodèse rachidienne, mais présente l'inconvénient de rendre parfois très difficile l'alignement de la barre de liaison par rapport aux vertèbres.

La présente invention a ainsi pour but de réaliser un dispositif d'immobilisation d'une barre par rapport à une partie osseuse en vue, par exemple, de réaliser une ostéosynthèse ou une arthrodèse, notamment rachidienne, qui obvie aux inconvénients mentionnés ci-dessus.

Plus précisément, la présente invention a pour objet un dispositif d'immobilisation d'une barre par rapport à une partie osseuse en vue, par exemple, de réaliser une ostéosynthèse ou une arthrodèse, notamment rachidienne, comportant des moyens de fixation, ces moyens comportant une tige et une tête à deux faces sensiblement opposées, ladite tige étant solidaire de l'une des deux faces de ladite tête et apte à être liée directement ou indirectement à ladite partie osseuse, un cavalier en forme sensiblement de "U" comprenant au moins une base et deux branches latérales solidaires de ladite base, deux crochets montés respectivement sur l'extrémité libre des deux branches latérales, lesdits crochets étant montés latéralement aux extrémités libres des deux dites branches, vers l'intérieur dudit "U" et sensiblement en regard l'un de l'autre, et des moyens pour exercer un couple de forces opposées entre ledit cavalier et ladite tête par l'intermédiaire de ladite barre de façon à solidariser ensemble ledit cavalier, ladite tête et ladite barre,
caractérisé par le fait que la distance séparant les deux dits crochets est inférieure à la largeur d'au moins une partie de ladite tête et supérieure à la section de ladite tige et de ladite barre, la distance séparant intérieurement les deux dites branches latérales et la profondeur dudit "U" étant supérieures à la section de ladite barre.

D'autres caractéristiques et avantages de la présente invention apparaîtront au cours de la description suivante donnée en regard des dessins annexés à titre illustratif, mais nullement limitatif, dans lesquels:
la figure 1 représente une vue en perspective éclatée d'un mode de realisation du dispositif selon l'invention pour immobiliser une barre pour ostéosynthèse ou arthrodèse, notamment rachidienne.
La figure 2 représente une coupe schématique du mode de réalisation selon la figure 1, mais avec tous les éléments constitutifs du dispositif assemblés,
La figure 3 représente un schéma permettant d'expliciter l'utilisation et les avantages du dispositif selon l'invention, et
La figure 4 représente, en coupe et en transparence, un autre mode de réalisation d'une partie du dispositif selon l'invention.

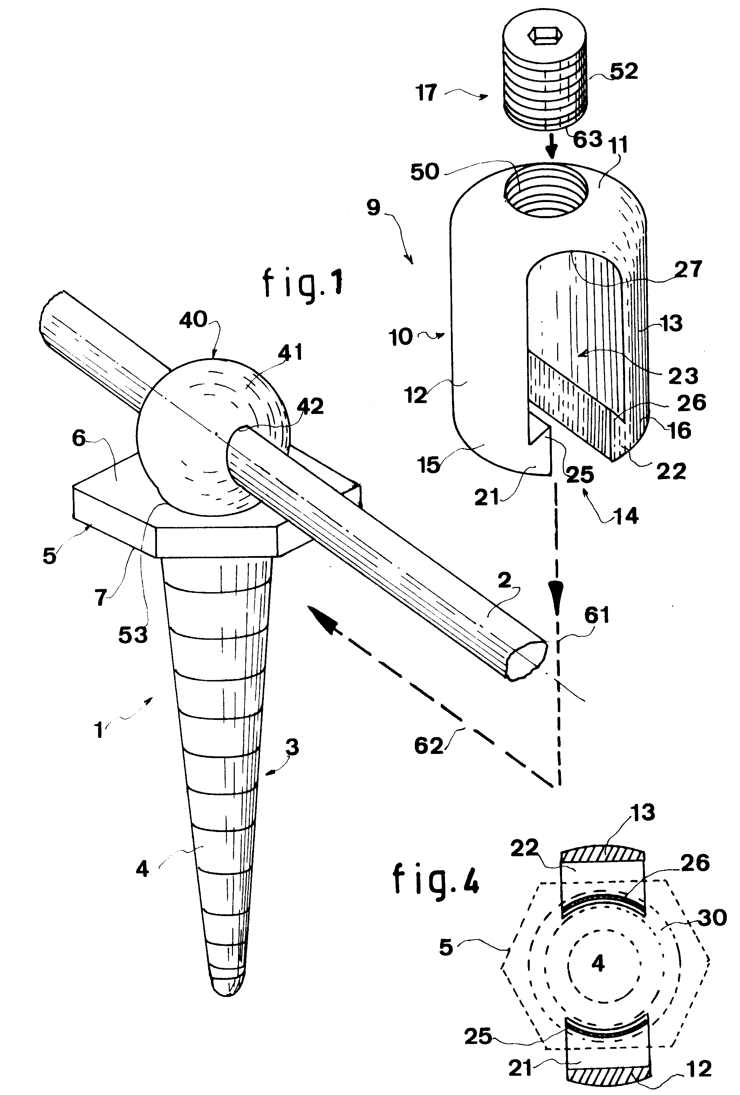

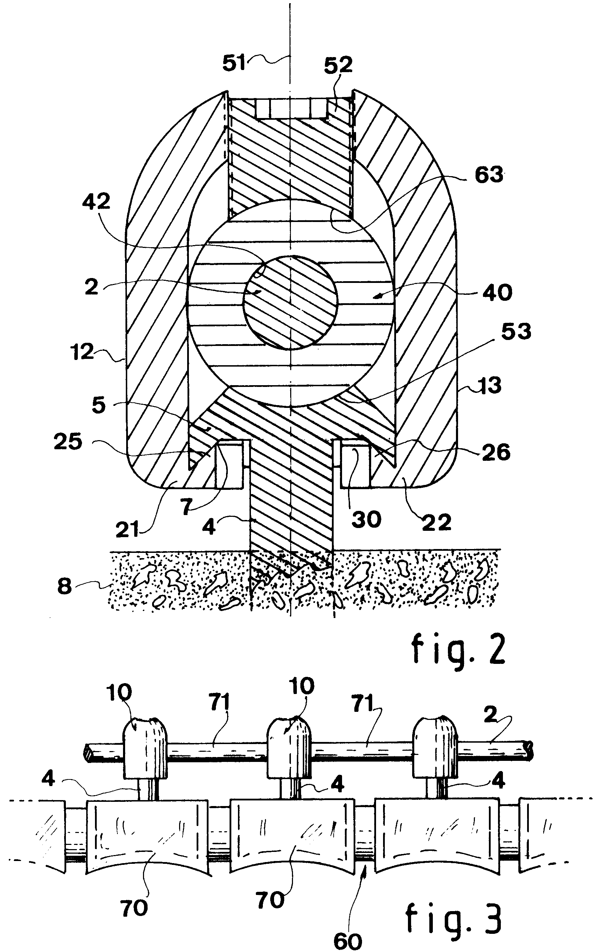

Les figures 1 et 2 représentent un mode de réalisation d'un dispositif 1 pour immobiliser une barre 2 pour, notamment, ostéosynthèse ou arthrodèse rachidienne. Ce dispositif comporte des moyens de fixation 3 sur une partie osseuse 8, ces moyens comprenant une tige 4 et une tête 5 à deux faces 6, 7 sensiblement opposées, la section de la tête étant supérieure à la section de la tige, la tige 4 est solidaire de l'une 7 des deux faces de la tête et elle est apte à être liée directement ou indirectement à la partie osseuse 8, par exemple par vissage quand elle est filetée ou par tout autre moyen adapté. Le dispositif comporte en outre des moyens 9 pour solidariser la barre 2 avec la tête 5.

Ces moyens 9 pour solidariser la barre 2 avec la tête 5 comprennent un cavalier 10 en forme sensiblement de "U" comprenant au moins une base 11 et au moins deux branches latérales 12, 13 solidaires de cette base 11, des moyens d'accrochage 14 de l'extrémité libre 15, 16 des deux branches latérales 12, 13 du cavalier 10 non solidaire de la base 11, avec la tête 5, et des moyens 17 pour exercer un couple de forces opposées entre le cavalier 10 et la tête 5 par l'intermédiaire de la barre 2 de façon à solidariser ensemble le cavalier 10, la tête 5 et la barre 2.

Dans un mode de réalisation avantageux, les moyens d'accrochage 14 de l'extrémité libre 15, 16 des deux branches latérales 12, 13 du cavalier 10 non solidaire de la base 11, avec la tête 5, comportent deux crochets 21, 22 montés respectivement sur l'extrémité libre 15, 16 des deux branches latérales 12, 13. Ces crochets 21, 22 sont montés latéralement aux extrémités libres 15, 16 vers l'intérieur 23 du "U" et sensiblement en regard l'un de l'autre, la distance les séparant étant inférieure à la largeur d'au moins une partie de la tête 5 et supérieure à la section de la tige 4 et de la barre 2, la distance séparant intérieurement les deux branches latérales 12, 13 et la profondeur 23 du "U" étant supérieures à la section de la barre 2, cette section étant constituée par le diamètre de la barre dans le cas le plus avantageux où cette barre est cylindrique de révolution.

De plus, pour que la tête 5 puisse être introduite entre les deux branches du cavalier avant d'être solidarisée avec leur extrémité libre et pour que le cavalier 10 et la tête 5 puissent au moins légèrement pivoter l'un par rapport à l'autre sensiblement autour de l'axe de la tige 4, au moins une partie de la tête, sinon la totalité, a une largeur, prise suivant une direction perpendiculaire à l'axe de la tige 4, inférieure à la distance séparant intérieurement les deux branches latérales du cavalier.

Avantageusement, les moyens d'accrochage 14 de l'extrémité libre 15, 16 des deux branches latérales 12, 13 du cavalier 10, avec la tête 5, comportent en outre une partie en saillie 25, 26 sur chacun des crochets 21, 22, ces parties 25, 26 formant saillie vers le fond 27 du "U". Dans ce cas, pour obtenir une bonne association des crochets avec la tête 5, cette tête comporte avantageusement une rainure en creux 30 réalisée sur sa face 7 solidaire de la tige 4 et d'une section sensiblement complémentaire de celle des parties en saillie 25, 26.

Dans une réalisation avantageuse, cette rainure 30 et les parties en saillie 25, 26 sont définies sur deux lignes sensiblement circulaires de même rayon, comme représenté sur la figure 4, de façon à obtenir un engagement parfait des parties en saillie 25, 26 dans la rainure 30 quelles que soient les positions angulaires relatives de la tête 5 et du cavalier 10.

De plus, comme cela apparaît sur la figure 2, la section de la rainure 30 et des parties en saillie 25, 26 est avantageusement triangulaire ou d'une forme pouvant être assimilée à une forme triangulaire ou analogue, pour obtenir un engagement du type conique et favoriser un couplage solide et rigide entre la tête 5 et le cavalier 10.

Il est précisé que l'exemple du cavalier 10 illustré notamment sur la figure 1 est une réalisarion préférentielle pour la plupart des cas d'utilisation du dispositif. Cependant, sans pour autant perdre sa forme sensiblement en "U", le cavalier peut être réalisé de façon que ses deux branches latérales 12, 13 soient reliées entre elles sur l'une des deux faces du "U" par une paroi à proximité des extrémités 15, 16 non solidaires de la base 11. 11 est seulement nécessaire que, dans cette paroi, soit définie une ouverture qui permette le passage de la barre 2 et la translation du cavalier 10 suivant son axe 51 pour que les moyens d'accrochage 14 puissent coopérer avec la tête 5.

Dans un mode de réalisation préférentiel, comme illustré sur les figures 1 et 2, les moyens 17 pour exercer un couple de forces opposées entre le cavalier 10 et la tête 5 par l'intermédiaire de la barre 2 de façon à solidariser ensemble le cavalier 10, la tête 5 et la barre 2 comportent un élément de liaison 40 entourant au moins partiellement la barre 2 et avantageusement déformable. Lorsque les différents éléments constitutifs du dispositif sont assemblés, cet élément de liaison 40 est situé entre les deux branches latérales 12, 13 du cavalier 10 et repose sur la face 6 de la tête 5 opposée à la face 7 solidaire de la tige 4. De préférence, cet élément de liaison 40 affecte, par sa surface extérieure 41, une forme sensiblement sphérique d'un diamètre légèrement inférieur à la distance séparant intérieurement les deux branches latérales 12, 13 et comporte une percée diamétrale 42 d'une section sensiblement égale ou légèrement supérieure à la section de la barre 2. il peut être constitué, par exemple, par une olive fendue et/ou réalisée en un matériau élastique.

Pour favoriser le centrage de la barre 2 par rapport aux deux branches latérales 12, 13, la face 6 de la tête opposée à la face 7 solidaire de la tige 4 comporte un logement en creux 53 complémentaire d'au moins une portion de la surface extérieure 41 sensiblement sphérique de l'élément de liaison 40.

Les moyens 14 pour exercer un couple de forces opposées entre le cavalier 10 et la tête 5 par l'intermédiaire de la barre 2 comportent en outre, par exemple comme illustré, un orifice taraudé 50 traversant la base 11 du cavalier 10 suivant un axe 51 sensiblement parallèle aux deux branches latérales 12, 13 et un écrou fileté 52 au même pas que l'orifice taraudé 50, l'écrou étant apte à être vissé dans cet orifice 50 de façon à venir en direction de la barre 2 quand elle est placée entre les deux branches 12, 13 du cavalier et à venir au contact de l'élément de liaison 40.

Quand l'élément de liaison 40 est sphérique, la face 63 de l'écrou 52 qui vient à son contact est concave sphérique, complémentaire de la surface extérieure de l'élément 40, pour que la force pressante exercée par l'écrou 52 sur l'élément de liaison soit parfaitement répartie sur une surface la plus grande possible.

Il est précisé que la tige 4 peut être une tige filetée apte à se placer directement par vissage dans une partie d'os, mais qu'elle peut être aussi une tige de liaison indirecte entre deux parties osseuses au moyen, par exemple, de deux barres qui sont elles-mêmes fixées à ces parties osseuses par tous moyens, notamment par des dispositifs selon l'invention.

De même, le cavalier peut ne comporter qu'une base et deux branches latérales 12, 13 pour immobiliser une barre 2. Mais il peut aussi comporter, sur une même base 11, trois branches permettant de définir deux "U" juxtaposés, par exemple pour immobiliser ensemble deux barres 2.

Le dispositif d'immobilisation d'une barre pour ostéosynthèse ou arthrodèse décrit ci-dessus s'utilise de la façon suivante décrite dans le cas d'une arthrodèse rachidienne schématiquement représentée, figure 3, sur une colonne vertébrale 60.

Le chirurgien commence par placer plusieurs tiges 4 dans les vertèbres 70 en les vissant jusqu'à obtenir leur enfoncement limite défini, par exemple, par une butée sur chaque tige 4. Sur la barre 2, il enfile autant d'éléments de liaison 40 qu'il y a de têtes 5 qui emergent des vertèbres 70 et donne à la barre la forme voulue pour qu'elle passe par toutes les têtes 5 et que les éléments de liaison 40 puissent reposer dans les logements 53 prévus à cet effet sur leur face 6. La barre et les éléments de liaison se trouvent alors dans la position représentée sur la partie gauche de la figure 1. Le chirurgien prend ensuite autant de cavaliers 10 que de têtes apparentes et les place en coopération avec ces têtes, comme décrit ci-après.

Chaque cavalier est glissé sur la barre, par exemple dans l'espace 71 compris entre deux têtes, de façon que ses deux branches latérales encadrent "à cheval" la barre, celle-ci se trouvant positionnée dans le creux 23 du "U" et avantageusement plaquée contre le fond 27. Ce premier mouvement de chaque cavalier est illustré par la portion de droite en traits interrompus 61 sur la figure 1. A partir de cette position, le cavalier est translaté suivant le trajet illustré, sur la figure 1, par la portion de droite en traits interrompus 62, jusqu'à ce que les parties en saillie 25, 26 viennent s'engager dans la rainure 30 de la tête à laquelle ce cavalier doit être associé. De par la forme de la section de ces parties en saillie et de la rainure, les parties en saillie viennent s'enficher en force dans la rainure par un coincement dit conique, et ce, quelle que soit la position angulaire de la tête puisque, comme décrit ci-avant, cette rainure 30 et les parties en saillie 25, 26 sont circulaires de même rayon.

Le chirurgien peut alors placer l'écrou 52 en le vissant dans l'orifice taraudé 50, jusqu'à ce que son extrémité 63 vienne au contact de l'élément de liaison 40 et appuie fortement sur lui pour que, par réaction, il s'exerce un couple de forces entre le cavalier et la tête capable de maintenir parfaitement solidaires le cavalier 10, la tête 5 et la barre 2 par l'intermédiaire de l'élément de liaison 40.

A la description ci-dessus du mode d'assemblage des éléments constitutifs du dispositif d'immobilisation d'une barre pour ostéosynthèse ou arthrodèse selon l'invention, il est évident que ce dispositif évite tous les inconvénients mentionnés au préambule tout en conservant les avantages des dispositifs de l'art antérieur.

## Revendications

**1-** Dispositif d'immobilisation (1) d'une barre (2) par rapport à une partie osseuse en vue, par exemple, de réaliser une ostéosynthèse ou une arthrodèse, notamment rachidienne, comportant:
- des moyens de fixation (3), ces moyens comportant une tige (4) et une tête (5) à deux faces (6,7) sensiblement opposées, ladite tige étant solidaire de l'une des deux faces de ladite tête et apte à être liée directement ou indirectement à ladite partie osseuse (8),
- un cavalier (10) en forme sensiblement de "U" comprenant au moins une base (11) et deux branches latérales (12,13) solidaires de ladite base,
- deux crochets (21,22) montés respectivement sur l'extrémité libre des deux branches latérales, lesdits crochets (21,22) étant montés latéralement aux extrémités libres des deux dites branches, vers l'intérieur (23) dudit "U" et sensiblement en regard l'un de l'autre, et
- des moyens (17) pour exercer un couple de forces opposées entre ledit cavalier (10) et ladite tête (5) par l'intermédiaire de ladite barre (2) de façon à solidariser ensemble ledit cavalier, ladite tête et ladite barre, caractérisé par le fait que la distance séparant ces deux dits crochets est inférieure à la largeur d'au moins une partie de ladite tête (5) et supérieure à la section de ladite tige (5) et de ladite barre (2), la distance séparant intérieurement les deux dites branches (12,13) latérales et la profondeur dudit "U" étant supérieures à la section de ladite barre (2).

**2-** Dispositif selon la revendication 1, caractérisé par le fait que lesdits moyens d'accrochage (14) de l'extrémité libre (15,16) des deux branches latérales (12,13) non solidaire de ladite base (11), avec ladite tête (5), coniportent en outre une partie en saillie (25,26) sur chacun desdits crochets, lesdites parties formant saillie vers le fond (27) dudit "U".

**3-** Dispositif selon la revendication 2, caractérisé par le fait que la face (7) de ladite tête (5) solidaire de ladite tige (4) comporte une rainure (30) en creux dont la section est sensiblement complémentaire de celle desdites parties en saillie (25,26).

**4-** Dispositif selon la revendication 3, caractérisé par le fait que ladite rainure (30) et lesdites parties en saillie (25,26) sont définies sur deux lignes sensiblement circulaires de même rayon.

**5-** Dispositif selon la revendication 4, caractérisé par le fait que la section de ladite rainure (30) et des parties en saillie (25,26) est triangulaire.

**6-** Dispositif selon l'une des revendications 2 à 5, caractérisé par le fait que les moyens (17) pour exercer un couple de forces opposées entre ledit cavalier (10) et ladite tête (5) par l'intermédiaire de ladite barre (2) de façon à solidariser ensemble ledit cavalier, ladite tête et ladite barre comportent un élément de liaison (40) entourant au moins partiellement ladite barre (2), ledit élément de liaison reposant sur la face (6) de la tête (5) opposée à la face (7) solidaire de la tige (4) et étant situé entre les deux branches latérales (12,13) du cavalier (10).

**7-** Dispositif selon la revendication 6, caractérisé par le fait que ledit élément de liaison (40) affecte, par sa surface extérieure (41), une forme sensiblement sphérique et comporte une percée diamétrale (42) d'une section au moins égale à la section de ladite barre (2), cedit élément de liaison étant déformable.

**8-** Dispositif selon la revendication 7, caractérisé par le fait que ledit élément de liaison déformable est constitué par une olive comportant au moins l'une des caractéristiques suivantes: fendue, réalisée en un matériau élastique.

**9-** Dispositif selon l'une des revendications 1 à 8, caractérisé par le fait que les moyens (17) pour exercer un couple de forces opposées entre ledit cavalier (10) et ladite tête (5) par l'intermédiaire de ladite barre (2) de façon à solidariser ensemble ledit cavalier (10), ladite tête (5) et ladite barre (2) comportent un orifice taraudé (50) traversant la base (11) dudit cavalier en "U" suivant un axe (51) sensiblement parallèle aux branches latérales et un écrou (52) fileté au même pas que ledit orifice taraudé, ledit écrou étant apte à être vissé dans ledit orifice de façon à venir en direction de ladite barre (2) quand elle est placée entre les deux dites branches latérales du "U" et à venir au contact dudit element de liaison (40).

**10 -** Dispositif selon les revendications 7 et 9, caractérisé par le fait que la face (63) dudit écrou (52) venant au contact dudit élément de liaison (40) est une surface concave sensiblement sphérique.

**11-** Dispositif selon l'une des revendications 7 à 10, caractérisé par le fait que la face (6) de ladite tête (5) opposée à celle (7) qui est solidaire de la tige (4) comporte un logement en creux (53) complémentaire d'au moins une portion de la surface extérieure sensiblement sphérique dudit élément de liaison (40).

**12 -** Dispositif selon l'une des revendications 1 à 11, caractérisé par le fait qu'au moins une partie de ladite tête (5) a une largeur, prise suivant une direction sensiblement perpendiculaire à l'axe de la tige (4), inférieure à la distance séparant intérieurement les deux branches latérales du cavalier.
